# FASCICULE DE BREVET EUROPEEN

(11) **EP 2 983 488 B1**
(45) Date de publication et mention de la délivrance du brevet: **12.05.2021**
(21) Numéro de dépôt: 14715360.5
(22) Date de dépôt: 12.03.2014
(51) Int. Cl.: A23G 1/48, A23G 3/34, A23G 1/30, A23G 4/06, A23L 5/00, A23L 7/00, A23L 9/00, A23L 21/00, A23L 5/42, A23L 5/40, A61K 9/28, A23P 20/00, A23P 20/10, C08K 5/101

(54) **COMPOSITION D'ENROBAGE A BASE D'ALIMENTS COLORANTS**
BESCHICHTUNGSZUSAMMENSETZUNG AUF BASIS VON LEBENSMITTELFARBSTOFFEN
COATING COMPOSITION BASED ON FOOD COLOURING AGENTS

(30) Priorité: 13.03.2013 FR 1352237
(43) Date de publication de la demande: 17.02.2016
(73) Titulaire: Societe D Exploitation De Produits Pour Les Industries Chimiques Seppic, 75007 Paris (FR)
(72) Inventeur: LEFEBVRE, Sandra, F-81100 Castres (FR); ROUANET, Philippe, F-81220 L'albarède Guitalens (FR)
(74) Mandataire: Air Liquide
(86) Numéro de dépôt international: PCT/FR2014/050565
(87) Numéro de publication internationale: WO 2014/140482

(56) Documents cités:
- EP-A1- 0 542 510
- EP-A1- 0 542 510
- EP-A1- 1 764 003
- US-A- 5 194 278
- US-A1- 2005 147 724
- US-A1- 2011 280 942

## Description

La présente invention a pour objet des compositions d'enrobage destinées à l'enrobage coloré de formes solides ingérables, et plus particulièrement des comprimés pharmaceutiques, vétérinaires ou compléments alimentaires, les procédés de préparation desdites compositions d'enrobage, les procédés d'enrobage mettant en œuvre lesdites compositions d'enrobage, et les produits enrobés colorés obtenus par la mise en œuvre des procédés d'enrobage comprenant l'utilisation desdites compositions d'enrobage.

Les formes sèches utilisées dans les industries de la pharmacie humaine ou vétérinaire, et des compléments alimentaires, se présentent généralement sous la forme de comprimés, de gélules, de dragées, de granulés, qui sont réalisés par agglomération de particules solides comprenant au moins un principe actif et/ou au moins un ingrédient nutritionnel et au moins un agent excipient. Ces formes sèches peuvent être préparées par la mise en œuvre de nombreuses techniques connues de l'homme du métier, comme par exemple les techniques de compression, de granulation, de compactage ou d'extrusion.

Afin de protéger ces formes sèches contre la dégradation générée par des conditions extérieures, comme par exemple la dégradation des principes actifs compris dans celles-ci par les rayonnements de la lumière, ou bien contre l'abrasion lors de leur conditionnement, ou bien contre la formation de poussières, et/ou afin d'améliorer l'aspect extérieur desdites formes sèches, en leur conférant une couleur spécifique ou une brillance améliorée, on recouvre lesdites formes sèches d'une pellicule d'une composition chimique agissant comme agent d'enrobage.

Les compositions d'enrobage sont bien connues de l'homme du métier et sont généralement constituées par au moins un agent filmogène et par un système colorant, constitué par des pigments d'origine minérale, comme par exemple les oxydes de fer, ou par des colorants.

Les colorants azoïques sont usuellement utilisés comme agents colorants dans la préparation d'une composition d'enrobage, pour conférer une couleur vive à la pellicule formée sur la forme sèche.

De récentes évolutions dans la règlementation européenne relative à l'usage et à l'utilisation des colorants azoïques amènent les industriels à rechercher une alternative et à mettre au point des systèmes de coloration de compositions d'enrobage conférant une couleur vive, stables à la lumière dans le temps, mais dénuées de dérivés azoïques.

Les colorants dits « naturels » peuvent constituer une alternative aux colorants azoïques. La plupart des colorants naturels sont obtenus par extraction sélective de molécules colorantes présentes dans des fruits et des légumes. On peut citer par exemple les anthocyanes, les caroténoïdes... Ils permettent d'obtenir des couleurs vives, mais la stabilité de ces couleurs, conférées aux formes sèches par ces colorants, à la lumière est souvent médiocre.

Se basant sur la directive européenne n° 1334/2008 qui définit les arômes naturels utilisés en alimentaire, l'association professionnelle NATCOL (Natural Food Colours Association) propose une classification des colorants naturels en fonction de leur mode d'obtention en transposant la directive aux colorants de manière à établir des définitions claires, ces mêmes définitions seront utilisées dans la présente demande de brevet.

Cette classification comporte deux classes : les colorants non présents dans la nature et les colorants présents dans la nature. Parmi ces derniers, la classification distingue les substances colorantes obtenues par synthèse chimique et les substances colorantes obtenues par la mise en œuvre de procédés utilisant comme matière première des sources naturelles comme par exemple des matières premières d'origine animale, végétale, minérale, mises en œuvre dans des procédés d'extraction et/ou de distillation et/ou de fermentation enzymatique.

Parmi ces colorants naturels présents dans la nature, et obtenus à partir de matières premières naturelles, on peut ainsi citer les anthocyanines (E163), les Chlorophylles (E 140), les caroténoïdes (E 160a), le curcumin (E100), la lutéine (E161b), le lycopène (E160b), les extraits de paprika (E160c).

Cependant, l'utilisation de ces colorants naturels, présents dans la nature, et obtenus à partir de matières premières naturelles, dans la préparation d'une composition de pelliculage ne permet pas de conférer à la forme sèche qui en est revêtue des couleurs stables lors d'une exposition prolongée à la lumière.

La demande de brevet WO2011/089248 divulgue des compositions comprenant :
a) une phase aqueuse comprenant de l'eau, au moins un co-solvant sélectionné parmi le groupe constitué par le propylène glycol, l'éthanol, la triacétine et le glycérol et optionnellement au moins un sucre sélectionné parmi le groupe constitué par le sucrose, le fructose et le glucose ;
b) un système tensio-actif comprenant au moins une saponine, et de la lécithine ; et
c) une phase huile comprenant au moins une substance sélectionnée parmi le groupe constitué par les arômes, les vitamines, les colorants, et les acides gras polyinsaturés.

Ces compositions sont utilisées pour préparer des compositions alimentaires (aliments et boissons).

La description de cette demande de brevet divulgue des colorants préférés : caraténoïdes, lutéines, extraits de paprika.

La demande de brevet US 2007/0202222 divulgue un boitier alimentaire comprenant un matériau textile et/ou de la cellulose régénérée, imprégnés ou enrobés dans leur partie intérieure par au moins un colorant.

Les aliments colorants décrits sont notamment le paprika, la chicorée, des épices, le caramel, des extraits de tomates.

La demande de brevet WO 2006/066389 décrit et revendique des compositions se présentant sous la forme de capsules sèches comprenant une matrice insoluble comprenant au moins 70% massique de protéines, entre 5 et 10% d'eau et un actif encapsulé, caractérisée en ce qu'elle présente, une fois mouillée dans une solution aqueuse incolore ou une huile minérale, une composante colorimétrique L* de clarté supérieure à 40, une valeur colorimétrique C* de saturation de couleur inférieure ou égale à 33 et un « angle de teinte » entre 70° et 90°. EP 0 542 510 A1 divulgue une composition d'enrobage comprenant poudre de curcuma et poudre de betterave, ainsi que d'amidon de maïs cireux pré-gélatinisé et d'amidon de maïs, dans l'eau.

US 5 194 278 A divulgue une composition d'enrobage compenant du xanthan et du jus concentré des raisins.

US 2011/0280942 A1 divulgue des compositions de revêtement comprenant un polymère cellulosique, un agent opacifiant et un acide gras. Les ingrédients facultatifs sont des colorants.

Les inventeurs de la présente invention ont donc cherché à mettre au point des compositions destinées à enrober des formes sèches, procurant un film coloré sur celles-ci dont la couleur est de forte intensité, laquelle reste stable après une durée prolongée d'exposition à la lumière, ces compositions d'enrobage devant être exemptés de colorants de type azoïques.

Selon un premier aspect, l'invention a pour objet une composition d'enrobage selon la revendication 1, comprenant pour 100% de sa masse: de 10% à 90% massique de deux polymères filmogènes : l'Hydroxy Propyl Méthyl Cellulose, et la Carboxy Méthyl Cellulose de sodium, de 10 % à 40% massique d'au moins un aliment colorant, et de 0% à 50% massique d'au moins un agent auxiliaire d'enrobage choisi parmi les opacifiants de couleur blanche, les diluants, les tensioactifs, les plastifiants, les anti-mousses.

Selon un aspect particulier, l'invention a pour objet une composition telle que définie ci-dessus, caractérisée en ce que ledit au moins un agent auxiliaire d'enrobage est choisi parmi les opacifiants de couleur blanche tels que le dioxyde de titane, le talc, le kaolin, l'oxyde de magnésium ; les diluants tels que le lactose, le sucrose, le mannitol, le sorbitol, le xylose, le xylitol, l'isomalt, la cellulose, le talc, les amidons natifs ; les tensioactifs tels que les esters de sorbitan, les esters de sorbitan éthoxylés, les huiles de ricin hydrogénées et éthoxylées, les lécithines, le sodium lauryl sulfate, les alcools gras éthoxylés, les acides gras éthoxylés ; les plastifiants tels que la glycérine, les polypropylène glycols, les polyéthylènes glycols ou leur dérivés de condensation avec un acide gras ou un alcool gras, l'acide stéarique et ses dérivés, les mono glycérides acétylés ; les anti-mousses tels que les acides gras ou les dérivés de silicone.

Selon un aspect particulier, l'invention a pour objet une composition telle que définie ci-dessus, caractérisée en ce que ledit au moins un aliment colorant est choisi parmi les fruits et les légumes concentrés, comme par exemple le carthame, les algues, la carotte, la carotte pourpre, la carotte noire, l'hibiscus, le radis, le cassis, la pomme, le citron, les épinards, les raisins, le cassis, le choux rouge, les baies de sureau, la betterave, la citrouille, le potiron, les orties, le curcuma, le safran, les fruits rouges, le paprika.

Selon un aspect particulier, l'invention a pour objet une composition telle que définie ci-dessus, caractérisée en ce qu'elle comprend de 10% à 40% massique d'aliments colorants. De préférence elle comprend de 10% à 30% massique d'aliments colorants.

Les colorants alimentaires (« food color »), qui nécessitent une approbation selon la réglementation européenne sont à distinguer des aliments colorants (« coloring foodstuff ») qui ne nécessitent pas cette approbation réglementaire et qui sont mis en œuvre dans la composition objet de la présente invention.

La définition du terme « aliment colorant » est établie en fonction du mode d'obtention dudit aliment. En effet, l'obtention d'un aliment colorant ne doit pas consister en une extraction sélective des substances colorantes, en outre l'aliment colorant doit conserver ses caractéristiques organoleptiques, à savoir son goût, sa saveur et sa couleur. Par exemple, une purée d'épinard résultant de la cuisson, du broyage, du séchage ou de la concentration des épinards mis en œuvre sous leur forme végétale, et utilisée pour procurer une couleur verte, est un aliment colorant.

Les aliments colorants sont des fruits et des légumes concentrés ayant la propriété de colorer. Ils ont le statut d'ingrédient et non d'additif. Ils présentent en particulier les propriétés suivantes :
- provenant de plantes comestibles,
- traitement physique utilisant uniquement l'eau comme solvant,
- pas d'extraction sélective de la matière colorante,
- pas d'ajout d'additif fonctionnel.

Dans l'état de la technique, rien ne divulgue, ni ne suggère l'emploi de compositions d'enrobage comprenant des aliments colorants en association avec hydroxy propyl méthyl cellulose et carboxy méthyl cellulose de sodium pour améliorer la stabilité de l'intensité de la couleur d'une forme solide revêtue desdites compositions d'enrobage (à destination d'utilisation alimentaire, vétérinaire ou pharmaceutique) lors d'une exposition prolongée à la lumière par rapport à une même forme solide revêtue d'une composition d'enrobage comprenant un colorant alimentaire ou un pigment coloré.

Or, de manière surprenante, l'emploi dans des compositions d'enrobage de ces fruits et légumes concentrés permet d'obtenir des couleurs vives plus stables que celles obtenues avec des compositions d'enrobage comprenant des colorants naturels extraits sélectivement des fruits et légumes.

Selon un autre aspect particulier, l'invention a pour objet une composition d'enrobage telle que définie précédemment, caractérisée en ce qu'elle se présente sous une forme prête à l'emploi contenant les mélanges de ses différents constituants sous la forme d'une dispersion aqueuse, d'une poudre ou de granulés prêts à l'emploi.

Par dispersion aqueuse, on entend les dispersions réalisées dans l'eau ou les mélanges d'eau et d'alcools hydrosolubles comme par exemple l'éthanol.

Les compositions prêtes à l'emploi présentent plusieurs avantages :
- La manipulation, le stockage, le contrôle d'un seul et unique produit.
- Une meilleure reproductibilité des couleurs et des performances.
- Une mise en dispersion plus aisée.

L'invention a aussi pour objet un procédé de préparation d'une composition d'enrobage telle que définie précédemment et se présentant sous la forme d'une poudre sèche, comprenant les étapes suivantes :
- une étape (a) de mélange des polymères filmogènes, de l'aliment colorant et, si nécessaire ou si désiré d'un ou plusieurs autres agents auxiliaires d'enrobage,
- une étape (b) optionnelle de broyage du mélange issue de l'étape (a), pour former ladite composition d'enrobage.

Dans le procédé tel que défini précédemment, pour la mise en œuvre de l'étape (a), l'ensemble des composants est ajouté de manière séquencée ou simultanée.

Le mélange est ensuite généralement effectué avec un mélangeur de poudres de type mélangeur à pâles, mélangeur par retournement ou mélangeur en « V ».

L'étape (b) du procédé tel que défini ci-dessus est par exemple effectuée au moyen d'un broyeur à couteaux ou d'un broyeur à broches de façon à obtenir une poudre finement divisée ou avec un dispositif de cryo-broyage généralement sous azote liquide.

Un tel dispositif permet d'optimiser la granulométrie finale de la composition d'enrobage.

L'invention a aussi pour objet un procédé de préparation d'une composition d'enrobage telle que définie précédemment et se présentant sous la forme de granulés prêts à l'emploi, comprenant les étapes suivantes :
- une étape (a1) de mouillage du mélange des polymères filmogènes, de l'aliment colorant et, si nécessaire ou si désiré d'un ou plusieurs autres agents auxiliaires d'enrobage, par une solution liante, afin d'obtenir une masse humide contenant de 30% à 60% d'eau,
- une étape (b1) de séchage de la masse humide obtenue à l'étape (a1), et si désiré ou si nécessaire,
- une étape (c1) de calibrage de la masse séchée obtenue à l'étape (b1) pour obtenir ladite composition d'enrobage.

Le procédé tel que défini ci-dessus est par exemple décrit dans les demandes de brevet français publiées sous les numéros FR 2 548 675 et FR 2 660 317 ou dans l'encyclopédie Kirk Othmer (3ème édition volume 17, page 281).

Par granulés, on entend principalement des agglomérats de plusieurs dizaines à plusieurs milliers de particules de matière, initialement individualisées, pouvant être de nature identique ou différente.

Les étapes (a1) et (b1) du procédé tel que défini ci-dessus, sont notamment effectuées dans un mélangeur-granulateur ou dans un lit fluidisé.

L'étape (c1) du procédé tel que défini ci-dessus, est notamment effectuée dans une étuve ou dans un lit fluidisé.

L'invention a aussi pour objet un procédé de préparation d'une composition d'enrobage telle que définie précédemment et se présentant sous la forme d'une dispersion aqueuse, comprenant les étapes suivantes :
- une étape (a2) de dispersion des polymères filmogènes, de l'aliment colorant et, si nécessaire ou si désiré d'un ou plusieurs autres agents auxiliaires d'enrobage dans une phase aqueuse,
- une étape (b2) de broyage du mélange issue de l'étape (a1), pour former ladite composition d'enrobage.

L'invention a aussi pour objet l'utilisation de la composition d'enrobage, telle que définie précédemment, pour enrober des formes solides ingérables.

Par forme solide ingérable, on désigne les formes solides ingérables par l'homme ou l'animal et quelle qu'en soit leur destination, qu'il s'agisse de médicaments, de compléments alimentaires, de formes à visée cosmétique, de confiserie ou de sucrerie. L'utilisation de la composition d'enrobage, telle que définie précédemment, est plus particulièrement destinée aux comprimés.

L'invention a aussi pour objet un procédé d'enrobage de formes solides comestibles, comprenant :
- une étape (a3) de dispersion du mélange contenant les polymères filmogènes, l'aliment colorant et, si nécessaire ou si désiré les autres agents auxiliaires d'enrobage, dans un solvant adapté tel qu'un milieu aqueux ;
- une étape (b3) de pulvérisation de la dispersion obtenue à l'étape (a3) sur des substrats solides à enrober.

Dans l'étape (a3) du procédé tel que défini ci-dessus, les différents constituants sont maintenus en dispersion à l'aide d'un agitateur et d'une turbine défloculeuse ou d'une pâle de type « bateau », tout en évitant la formation de mousse.

Dans l'étape (a3) du procédé tel que défini ci-dessus, la composition d'enrobage représente de 6% à 30% massique, plus particulièrement de 6% à 25% massique, et encore plus particulièrement de 6% à 20% massique pour 100% de la masse de ladite dispersion.

### - Préparation des dispersions

Les compositions d'enrobage sont mises en dispersion à 25% massique dans l'eau. Pour chaque composition, 600g de dispersion sont préparés : 150g de composition sont dispersés dans 450g d'eau purifiée à 25°C. La mise en dispersion est réalisée à l'aide d'un agitateur de laboratoire type Turbotest V2004 (commercialisé par la société Rayneri) et d'une turbine défloculeuse. La vitesse d'agitation est ajustée de manière à éviter d'incorporer de l'air dans la dispersion, ce qui permet de ne pas former de mousse. Après 45 minutes d'agitation, les dispersions sont prêtes.

### - Pelliculage

Les dispersions sont pulvérisées sur des comprimés placebos dans une turbine de pelliculage perforée de marque Driacoater 500 commercialisée par la société DRIAM ; la charge de noyaux dans la turbine est de 3 kg. Les conditions opératoires suivantes sont suivies : débit d'air=300 m³/h, température d'entrée de l'air de séchage = 55°C-60°C. La température des noyaux varie entre 36°C-38°C pendant l'opération de pelliculage. Un dépôt sec théorique de 3% est appliqué sur les comprimés.

La mise en œuvre de la composition d'enrobage, selon les procédés précédemment décrits, permet de préparer des comprimés pharmaceutiques, vétérinaires ou compléments alimentaires enrobés. Les exemples suivants illustrent l'invention sans toutefois la limiter.

### Exemples.

**Exemple** 1 : des compositions d'enrobage, dont les constituants sont exprimés en pourcentage massique, sont formulées comme suit :

| | Exemple comparatif : composition A | Composition de l'invention : composition B |
|---|---|---|
| Agent filmogène | Hydroxy Propyl Methyl Cellulose (HPMC): 69,8% Carboxy Methyl Cellulose (MCC) : 12,2% | Hydroxy Propyl Methyl Cellulose (HPMC): 69,8% Carboxy Methyl Cellulose (MCC) : 12,2% |
| Agent auxiliaire | Monoglycérides acétylés : 6% | Monoglycérides acétylés : 6% |
| Système colorant | 12% de colorant naturel : Caroténoïdes. (Poudre de beta carotène : Fusion Red Orange P-WD. N°CAS est 7235-40-7) | 12% d'aliment colorant : Carottes noires |

Des comprimés de 500mg comprenant, pour 100% de leur masse, 49,75% massique de cellulose microcristalline, 49,75% massique de lactose et 0,5% massique de stéarate de magnésium sont enrobés avec les compositions d'enrobage A et B.

Les enrobages sont réalisés dans une turbine perforée de marque « turbine laboratoire PILOT XT » commercialisée par la société Profile Automation chargée à 2kg de comprimés.

A l'issue de l'étape précédente d'enrobage, les comprimés enrobés obtenus sont ensuite placés à la lumière du jour et à l'obscurité afin de mesurer la stabilité de la coloration.

La couleur des comprimés est contrôlée régulièrement grâce à un colorimètre de marque MEDSCAN, commercialisée par la société NEWTONE.

| Composition enrobant le comprimé | L*a*b* des comprimés à T=0 | L*a*b* des comprimés après 2 semaines à la lumière | L*a*b* des comprimés après 5 semaines à la lumière | L*a*b* des comprimés après 7 semaines à la lumière |
|---|---|---|---|---|
| Composition A | 64,4/ 57,9/ 59,8 | 74,4/ 25,9/ 67,2 | 92,9/ -0,4/ 4,6 (décoloration totale du comprimé) | Stabilité arrêtée |
| Composition B | 42,4/ 33,6/ - 0,8 | 44,5/ 36,0/ 0,0 | 48,2/ 34,5/ 0,0 (couleur des comprimés très proche de T=0) | 50,3/ 32,2/ 0,0 (couleur des comprimés proche de T=0) |

Interprétation en terme de saturation de couleur, selon le paramètre de saturation C*, calculé selon la formule : C*= (a*2+b*2))^{1/2}.

| Composition enrobant le comprimé | Saturation C* à T=0 | Saturation C* après 2 semaines à la lumière | Saturation C* après 5 semaines à la lumière | Saturation C* après 7 semaines à la lumière |
|---|---|---|---|---|
| Composition A | 83,2 | 72,0 | 4,6 | Stabilité arrêtée |
| Composition B | 33,6 | 36,0 | 34,5 | 32,2 |

La composition d'enrobage B comprenant l'aliment colorant permet d'obtenir des comprimés dont la couleur reste intense et stable pendant au moins 7 semaines à la lumière du jour.

A l'opposé, on observe une décoloration importante des comprimés enrobés par la composition A comprenant les caroténoïdes.

**Exemple** 2 : des compositions d'enrobage, dont les constituants sont exprimés en pourcentage massique, plus concentrées en colorants/aliments colorants ont été préparées et sont formulées comme suit :

| | Exemple comparatif : composition C | Exemple comparatif : composition D | Composition de l'invention : composition E |
|---|---|---|---|
| Agent filmogène | Hydroxy Propyl Methyl Cellulose (HPMC): 59%. Carboxy Methyl Cellulose (MCC) : 11%. | Hydroxy Propyl Methyl Cellulose (HPMC): 59%. Carboxy Methyl Cellulose (MCC) : 11%. | Hydroxy Propyl Methyl Cellulose (HPMC): 59%. Carboxy Methyl Cellulose (MCC) : 11%. |
| Coloration | 30% de colorant naturel : Caroténoïdes. (Poudre de beta carotène : Fusion Red Orange P-WD. N°CAS : 7235-40-7) | 30% de colorant naturel : Caroténoïdes. (Natrocol BETA CAROTENE WSP N°CAS : 7235-40-7) | 30% d'aliments colorants : Carottes pourpres |

Des comprimés de 500mg comprenant, pour 100% de leur masse, 49,75% massique de cellulose microcristalline, 49,75% massique de lactose et 0,5% massique de stéarate de magnésium sont enrobés avec les compositions d'enrobage C, D et E. Les enrobages sont réalisés dans une turbine perforée de marque « turbine laboratoire PILOT XT » commercialisée par la société Profile Automation chargée à 2kg de comprimés.

A l'issue de l'étape précédente d'enrobage, les comprimés enrobés obtenus sont ensuite placés en stabilité à la lumière du jour et à l'obscurité. La couleur des comprimés est contrôlée régulièrement grâce à un colorimètre de marque MEDSCAN, commercialisée par la société NEWTONE.

| Composition enrobant le comprimé | L*a*b* des comprimés à T=O | L*a*b* des comprimés après 2 semaines à la lumière | L*a*b* des comprimés après 5 semaines à la lumière | L*a*b* des comprimés après 5 semaines à l'obscurité | L*a*b* des comprimé s après 6 mois à la lumière | L*a*b* des comprimés après 6 mois à l'obscurité |
|---|---|---|---|---|---|---|
| Composition C | 58,8/ 53,1/ 52,5 | 67,6/ 47,2/ 55,2 | 84,8/ -1,5/ 65,3 (décoloration du comprimé) | 66.3/ 51.4/ 63.1 | Stabilité arrêtée | Stabilité arrêtée |
| Composition D | 67,8/ 46,9/ 65,1 | 75,5/ 19,6/ 74,0 | 84,4/ -3,7/ 75,1 (décoloration du comprimé) | 75.0/ 21.3/ 75.1 | Stabilité arrêtée | Stabilité arrêtée |
| Composition E | 58.7/ 50.7/ 28.2 | 62.3/ 46.5/ 23.8 | 62.2/ 46.0/ 19.0 | 62.8/ 48.1/ 22.8 | 66.7/ 42.3/ 19.2 | 62.8/ 47.5/ 21.3 |

Comme précédemment, la composition d'enrobage E selon l'invention, comprenant l'aliment colorant, permet d'obtenir une couleur des comprimés intense et stable pendant 6 mois à la lumière du jour. De plus, les paramètres L*a*b* mesurés après 6 mois à la lumière pour les comprimés enrobés avec la Composition E, montrent peu de différence avec les paramètres L*a*b* mesurés après 6 mois à l'obscurité.

A l'opposé, on observe rapidement la décoloration des comprimés enrobés avec les compositions C et D, comprenant les caroténoïdes comme système colorant. Les résultats ne sont donc pas dépendants de la dose de colorant utilisée.

Les paramètres utilisés pour décrire une couleur peuvent être regroupés en trois catégories :
- la tonalité chromatique qui caractérise la couleur en elle-même (vert, rouge...) ;
- la clarté qui caractérise la capacité de l'échantillon coloré à renvoyer plus ou moins la lumière (couleur claire, foncée...) ;
- la saturation qui caractérise l'intensité en couleur de l'échantillon (couleur vive = une couleur saturée).

Le système CIELAB 1976 est utilisé pour représenter mathématiquement les coordonnées colorimétriques d'une couleur.

Ce système possède deux modes de représentation :
- Représentation en coordonnées L*a*b* où L* représente l'axe de clarté ; a* l'axe Rouge/vert ; et b* l'axe Jaune/bleu.
- Représentation en coordonnées L*C*h0 où L* représente l'axe de clarté ; C* l'axe de la saturation ; h0 l'angle de teinte.

Pour qu'une couleur soit stable, il faut que la variation au cours du temps sur le L*a*b* ou le L*C*h0 soit la plus faible possible.

Les coordonnées L*a*b* ont été utilisées pour les exemples de la présente invention.

## Revendications

1. Composition d'enrobage comprenant pour 100% de sa masse :
- de 10% à 90% massique de deux polymères filmogènes : l'Hydroxy Propyl Methyl Cellulose, et la Carboxy Methyl Cellulose de sodium,
- de 10% à 40% massique d'au moins un aliment colorant, et
- de 0% à 50% massique d'au moins un agent auxiliaire d'enrobage choisi parmi les opacifiants de couleur blanche, les diluants, les tensioactifs, les plastifiants, les anti-mousses.

2. Composition d'enrobage selon la revendication précédente, **caractérisée en ce que** ledit au moins un agent auxiliaire d'enrobage est choisi parmi les opacifiants de couleur blanche tels que le dioxyde de titane, le talc, le kaolin, l'oxyde de magnésium ; les diluants tels que le lactose, le sucrose, le mannitol, le sorbitol, le xylose, le xylitol, l'isomalt, la cellulose, le talc, les amidons natifs ; les tensioactifs tels que les esters de sorbitan, les esters de sorbitan éthoxylés, les huiles de ricin hydrogénées et éthoxylées, les lécithines, le sodium lauryl sulfate, les alcools gras éthoxylés, les acides gras éthoxylés ; les plastifiants tels que la glycérine, les polypropylène glycols, les polyéthylènes glycols ou leur dérivés de condensation avec un acide gras ou un alcool gras, l'acide stéarique et ses dérivés, les mono glycérides acétylés ; les anti-mousses tels que les acides gras ou les dérivés de silicone.

3. Composition d'enrobage telle que définie à l'une des revendications précédentes, **caractérisée en ce que** ledit au moins un aliment colorant est choisi parmi les fruits et les légumes concentrés, comme par exemple le carthame, les algues, la carotte, la carotte pourpre, la carotte noire, l'hibiscus, le radis, le cassis, la pomme, le citron, les épinards, les raisins, le cassis, le choux rouge, les baies de sureau, la betterave, la citrouille, le potiron, les orties, le curcuma, le safran, les fruits rouges, le paprika.

4. Composition d'enrobage telle que définie à l'une des revendications précédentes, **caractérisée en ce qu'**elle comprend de 10% à 30% massique d'aliments colorants.

5. Composition d'enrobage telle que définie à l'une des revendications précédentes, **caractérisée en ce qu'**elle se présente sous la forme d'une dispersion aqueuse, d'une poudre ou de granulés prêts à l'emploi.

6. Procédé de préparation de la composition d'enrobage telle que définie à l'une des revendications 1 à 5, et se présentant sous la forme d'une poudre sèche, comprenant les étapes suivantes :
- une étape (a) de mélange du polymère filmogène, de l'aliment colorant et, si nécessaire ou si désiré d'un ou plusieurs autres agents auxiliaires d'enrobage,
- une étape (b) optionnelle de broyage du mélange issue de l'étape (a), pour former ladite composition d'enrobage.

7. Procédé de préparation d'une composition d'enrobage telle que définie l'une des revendications 1 à 5, et se présentant sous la forme de granulés prêts à l'emploi, comprenant les étapes suivantes :
- une étape (a1) de mouillage du mélange de polymère filmogène, de l'aliment colorant et, si nécessaire ou si désiré d'un ou plusieurs autres agents auxiliaires d'enrobage, par une solution liante, afin d'obtenir une masse humide contenant de 30% à 60% d'eau,
- une étape (b1) de séchage de la masse humide obtenue à l'étape (a1), et si désiré ou si nécessaire,
- une étape (c1) de calibrage de la masse séchée obtenue à l'étape (b1) pour obtenir ladite composition d'enrobage.

8. Procédé de préparation de la composition d'enrobage telle que définie à l'une des revendications 1 à 5, et se présentant sous la forme d'une dispersion aqueuse, comprenant les étapes suivantes :
- une étape (a2) de dispersion du polymère filmogène, de l'aliment colorant et, si nécessaire ou si désiré d'un ou plusieurs autres agents auxiliaires d'enrobage dans une phase aqueuse,
- une étape (b2) de broyage du mélange issue de l'étape (a1), pour former ladite composition d'enrobage.

9. Utilisation de la composition d'enrobage telle que définie aux revendications 1 à 5, pour enrober des formes solides ingérables.

10. Procédé de préparation de formes solides enrobées **caractérisé en ce qu'**il comprend :
- une étape (a3) de dispersion de la composition telle que définie à l'une des revendications 1 à 5 contenant le polymère filmogène, l'aliment colorant et, si nécessaire ou si désiré les autres agents auxiliaires d'enrobage, dans un solvant adapté tel qu'un milieu aqueux ;
- une étape (b3) de pulvérisation de la dispersion obtenue à l'étape (a3) sur des substrats solides à enrober.

## Patentansprüche

1. Überzugszusammensetzung, umfassend auf 100 % ihrer Masse:
- 10 % bis 90 % nach Masse an zwei filmbildenden Polymeren: Hydroxypropylmethylcellulose und Natriumcarboxymethylcellulose
- 10 % bis 40 % nach Masse an mindestens einem färbenden Lebensmittel, und
- 0 % bis 50 % nach Masse an mindestens einem Überzugshilfsstoff, der aus den weißfarbigen Deckmitteln, den Verdünnungsmitteln, den Tensiden, den Weichmachern, den Schaumverhütern ausgewählt ist.

2. Überzugszusammensetzung nach dem vorhergehenden Anspruch, **dadurch gekennzeichnet, dass** der mindestens eine Überzugshilfsstoff aus den weißfarbigen Deckmitteln wie etwa Titandioxid, Talkum, Kaolin, Magnesiumoxid; den Verdünnungsmitteln wie etwa Lactose, Saccharose, Mannit, Sorbit, Xylose, Xylit, Isomalt, Cellulose, Talkum, nativen Stärkesorten; den Tensiden sie etwa den Sorbitanestern, den ethoxylierten Sorbitanestern, den hydrierten und ethoxylierten Rizinusölen, den Lecithinen, Natriumlaurylsulfat, den ethoxylierten Fettalkoholen, den ethoxylierten Fettsäuren; den Weichmachern wie etwa Glycerin, den Polypropylenglykolen, den Polyethylenglykolen und deren Kondensationsderivaten mit einer Fettsäure oder einem Fettalkohol, Stearinsäure und deren Derivaten, den acetylierten Monoglyceriden; den Schaumverhütern wie etwa den Fettsäuren oder den Silikonderivaten ausgewählt ist.

3. Überzugszusammensetzung gemäß der Begriffsbestimmung in einem der vorhergehenden Ansprüche, **dadurch gekennzeichnet, dass** das mindestens eine färbende Lebensmittel aus den konzentrierten Früchten und Gemüsesorten wie beispielsweise Distel, Algen, Karotte, violetter Karotte, schwarzer Karotte, Hibiskus, Rettich, schwarzer Johannisbeere, Apfel, Zitrone, Spinat, Trauben, schwarzer Johannisbeere, Rotkohl, Fliederbeeren, Rübe, Gartenkürbis, Riesenkürbis, Brennnesseln, Kurkuma, Safran, roten Früchten, Paprika ausgewählt ist.

4. Überzugszusammensetzung gemäß der Begriffsbestimmung in einem der vorhergehenden Ansprüche, **dadurch gekennzeichnet, dass** sie 10 % bis 30 % nach Masse an färbenden Lebensmitteln umfasst.

5. Überzugszusammensetzung gemäß der Begriffsbestimmung in einem der vorhergehenden Ansprüche, **dadurch gekennzeichnet, dass** sie in Form einer wässrigen Dispersion, eines Pulvers oder eines gebrauchsfertigen Granulats vorliegt.

6. Verfahren zur Herstellung der Überzugszusammensetzung, welche der Begriffsbestimmung in einem der Ansprüche 1 bis 5 entspricht und in Form eines Trockenpulvers vorliegt, wobei es die folgenden Schritte umfasst:
- einen Schritt (a) des Vermischens des filmbildenden Polymers, des färbenden Lebensmittels sowie, sofern dies erforderlich ist oder gewünscht wird, eines oder mehrerer weiterer Überzugshilfsmittel,
- einen optionalen Schritt (b) des Zerkleinerns der Mischung, wie sie nach Abschluss des Schrittes (a) erhalten wurde, um die Überzugszusammensetzung zu bilden.

7. Verfahren zur Herstellung einer Überzugszusammensetzung, welche der Begriffsbestimmung in einem der Ansprüche 1 bis 5 entspricht und in Form eines gebrauchsfertigen Granulats vorliegt, wobei es die folgenden Schritte umfasst:
- einen Schritt (a1) des Benetzens der Mischung aus dem filmbildenden Polymer, dem färbenden Lebensmittel sowie, erforderlichenfalls oder wenn dies gewünscht wird, einem oder mehreren weiteren Überzugshilfsmitteln, mit einer bindend wirkenden Lösung, um eine feuchte Masse zu erhalten, die 30 % bis 60 % an Wasser enthält.
- einen Schritt (b1) des Trocknens der feuchten Masse, wie sie in Schritt (a1) erhalten wurde, sowie, wenn dies gewünscht wird oder erforderlich ist,
- einen Schritt (c1) des Kalibrieren der getrockneten Masse, wie sie in Schritt (b1) erhalten wurde, um die Überzugszusammensetzung zu erhalten.

8. Verfahren zur Herstellung der Überzugszusammensetzung, welche der Begriffsbestimmung in einem der Ansprüche 1 bis 5 und in Form einer wässrigen Dispersion vorliegt, wobei es die folgenden Schritte umfasst:
- einen Schritt (a2) des Dispergierens des filmbildenden Polymers, des färbenden Lebensmittels sowie, erforderlichenfalls oder wenn dies gewünscht wird, eines oder mehrerer weiterer Überzugshilfsmittel in einer wässrigen Phase,
- einen Schritt (b2) des Zerkleinerns der Mischung, wie sie nach Abschluss des Schrittes (a1) erhalten wurde, um die Überzugszusammensetzung zu bilden.

9. Verwendung der Überzugszusammensetzung gemäß der Begriffsbestimmung in einem der Ansprüche 1 à 5, um verzehrfähige feststoffliche Formen zu überziehen.

10. Verfahren zur Herstellung feststofflicher Formen mit Überzug, **dadurch gekennzeichnet, dass** es Folgendes umfasst:
- einen Schritt (a3) des Dispergierens der Zusammensetzung, welche der Begriffsbestimmung in einem der Ansprüche 1 bis 5 entspricht, wobei sie das filmbildende Polymer, das färbende Lebensmittel sowie, erforderlichenfalls oder wenn dies gewünscht wird, die weiteren Überzugshilfsstoffe enthält, in einem geeigneten Lösungsmittel wie etwa einem wässrigen Milieu;
- einen Schritt (b3) des Versprühens der Dispersion, wie sie im Schritt (a3) erhalten wurde, auf die zu überziehenden feststofflichen Substrate.

## Claims

1. Coating composition comprising, per 100% of the weight thereof:
- from 10 wt% to 90 wt% of two film-forming polymer: hydroxypropylmethyl cellulose and sodium carboxymethyl cellulose,
- from 10 wt% to 40 wt% of at least one coloring foodstuff, and
- from 0 to 50 wt% of at least one auxiliary coating agent selected from white opacifiers, diluents, surfactants, plasticizers and antifoams.

2. Coating composition according to the preceding claim, **characterized in that** said at least one auxiliary coating agent is selected from white opacifiers such as titanium dioxide, talc, kaolin, magnesium oxide; diluents such as lactose, sucrose, mannitol, sorbitol, xylose, xylitol, isomalt, cellulose, talc, native starches; surfactants such as sorbitan esters, ethoxylated sorbitan esters, hydrogenated and ethoxylated castor oils, lecithins, sodium lauryl sulfate, ethoxylated fatty alcohols, ethoxylated fatty acids; plasticizers such as glycerin, polypropylene glycols, polyethylene glycols or derivatives thereof from condensation with a fatty acid or fatty alcohol, stearic acid and derivatives thereof, acetylated monoglycerides; and antifoams such as fatty acids or silicone derivatives.

3. Coating composition as defined in one of the preceding claims, **characterized in that** said at least one coloring foodstuff is selected from concentrated fruit and vegetables, such as, for example, safflower, seaweed, carrot, purple carrot, black carrot, hibiscus, radish, blackcurrant, apple, lemon, spinach, grapes, blackcurrant, red cabbage, elderberries, beetroot, pumpkin, squash, nettles, turmeric, saffron, red berries and paprika.

4. Coating composition as defined in one of the preceding claims, **characterized in that** it comprises from 10 wt% to 30 wt% coloring foodstuffs.

5. Coating composition as defined in one of the preceding claims, **characterized in that** it is in the form of an aqueous dispersion, a powder or ready-to-use granules.

6. Process for preparing the coating composition as defined in one of Claims 1 to 5 and which is in the form of a dry powder, comprising the following steps:
- a step (a) of mixing the film-forming polymer, coloring foodstuff and, if necessary or desired, one or more other auxiliary coating agents,
- an optional step (b) of milling the mixture arising from step (a) to form said coating composition.

7. Process for preparing a coating composition as defined in one of Claims 1 to 5 and which is in the form of ready-to-use granules, comprising the following steps:
- a step (a1) of wetting the mixture of film-forming polymer, coloring foodstuff and, if necessary or desired, one or more other auxiliary coating agents using a binder solution, so as to obtain a wet mass containing from 30% to 60% water,
- a step (b1) of drying the wet mass obtained in step (a1) and, if desired or necessary,
- a step (c1) of calibrating the dried mass obtained in step (b1) to obtain said coating composition.

8. Process for preparing the coating composition as defined in one of Claims 1 to 5 which is in the form of an aqueous dispersion, comprising the following steps:
- a step (a2) of dispersing, in an aqueous phase, the film-forming polymer, the coloring foodstuff and, if necessary or desired, one or more other auxiliary coating agents,
- a step (b2) of milling the mixture arising from step (a1) to form said coating composition.

9. Use of the coating composition as defined in Claims 1 to 5 for coating ingestible solid forms.

10. Process for preparing coated solid forms, **characterized in that** it comprises:
- a step (a3) of dispersing, in a suitable solvent such as an aqueous medium, the composition as defined in one of Claims 1 to 5 containing the film-forming polymer, the coloring foodstuff and, if necessary or desired, the other auxiliary coating agents;
- a step (b3) of spraying the dispersion obtained in step (a3) onto solid substrates to be coated.
